# FASCICULE DE BREVET EUROPEEN

(11) **EP 0 403 360 B1**
(45) Date de publication et mention de la délivrance du brevet: **16.10.1996**
(21) Numéro de dépôt: 90401606.0
(22) Date de dépôt: 12.06.1990
(51) Int. Cl.: A61K 31/135, A61K 31/165, A61K 31/22

(54) **Utilisation de phényléthanolamines pour la préparation de médicaments pour les affections oculaires**
Verwendung von Phenylethanolaminen zur Herstellung von Medikamenten gegen Augenleiden
Use of phenylethanolamines for the preparation of a medicament for treating ophthalmologic disorders

(30) Priorité: 13.06.1989 FR 8907816; 13.06.1989 FR 8907817
(43) Date de publication de la demande: 19.12.1990
(73) Titulaire: SANOFI, 75008 Paris (FR); MIDY S.p.A., I-20137 Milano (IT)
(72) Inventeur: Manara, Luciano, I-15040 Pietra Marazzi, Alessandria (IT)
(74) Mandataire: Gillard, Marie-Louise

(56) Documents cités:
- EP-A- 0 040 915
- EP-A- 0 255 415
- "PHARMAKOLOGIE UND TOXIKOLOGIE", 1983, 4 édition, éditeur W. Forth et al., pages 94-95, B.I.-Wissenschaftsverlag;
- LIFE SCIENCES, vol. 35, no. 12, 17 septembre 1984, pages 1301-1309, Pergamon Press Ltd, US; S. WILSON et al.: "Genetically obese C57BL/6 ob/ob mice respond normally to sympathomimetic compounds"
- EUROPEAN JOURNAL OF PHARMACOLOGY, vol. 100, nos. 3-4, 1984, éditeur D. De Wied, pages 309-319, Elsevier Science Publishers B.V., Amsterdam, NL; C. WILSON et al.: "The rat lipolytic beta-adrenoceptor: Studies using novel beta- adrenopceptor agonists"
- JOURNAL OF CARDIOVASCULAR PHARMACOLOGY, vol. 6, no. 6, novembre/décembre 1984, pages 1216-1221, Raven Press, New York, US; C. WILSON et al.: "Beta- adrenoceptor subtypes in human, rat, guinea pig, and rabbit atria"
- FIDIA RESEARCH FOUNDATION SYMPOSIUM SERIES, vol. 2, 'Neurochemical Pharmacology', May 1989, pages 131-147, Raven Press, New York, US; L. MANNARA et al.: "New developments in beta-adrenergic mediated control of intestinal motility: Gut-specific phenylethanolaminotetralines"

## Description

La présente invention concerne l'utilisation de certains analogues de phényléthanolamines et de leurs sels pharmaceutiquement acceptables pour la préparation de compositions pharmaceutiques destinées au traitement des affections oculaires, en particulier au traitement de l'hypertension oculaire et du glaucome. La présente invention concerne également des compositions pharmaceutiques pour les mêmes indications.

Le glaucome est une affection oculaire caractérisée, entre autres symptomes, par une augmentation lente ou rapide de la pression intraoculaire. Le glaucome conduit à la destruction des fibres du nerf optique et peut entraîner la perte de la vision.

Un des traitements majeurs du glaucome consiste à réduire la pression intraoculaire. Les médicaments connus actuellement pour le traitement du glaucome présentent des difficultés d'emploi. Ainsi par exemple, la pilocarpine présente des effets secondaires locaux, tandis que des principes actifs comme l'épinéphrine ou un béta-bloquant adrénergique, le timolol, sont difficilement utilisables pour certains patients atteints de maladies cardiovasculaires ou ne supportant pas les effets cardiovasculaires généraux de ces drogues.

Les demandes de brevets européens publiées sous les n. 6735, 7204, 21636, 23385, 25331, 28105, 40000, 40915, 52963, 61907, 63004, 66351, 68669, 70133, 70134, 89154, 91749, 95827, 99707, 101069, 140359, 146392, 164700, 170121, 170135, 171519, le brevet belge 900.983, la demande de brevet UK 2133986, la demande de brevet australien publiée sous le n. 84/31944, la demande de brevet internationale publiée sous le n. WO 84/00956 et le brevet USA 4,391,826 décrivent des phényléthanolamines, éventuellement modifiées pour former des éthers, des esters ou des dérivés cycliques, ayant une activité antihyperglycémiante et/ou antiobésité.

La demande de brevet européen n. 255415 indique que les phényléthanolamines, leurs dérivés ou analogues décrits dans ces derniers brevets, ainsi que leurs sels, peuvent être utilisés pour la préparation de compositions pharmaceutiques actives sur le muscle lisse intestinal, destinées au traitement de troubles associés à une contraction du muscle lisse sans comporter d'effets secondaires cardiaques ou respiratoires importants, pourvu que lesdits produits ne contiennent pas de groupes hydroxy dans le noyau phényle de la phényléthanolamine.

Les demandes de brevets européens publiées sous les n. 211721, 303545 et 303546 et la demande de brevet européen n. 90 400 405.8 décrivent des phényléthanolamines caractérisées par un noyau tétralinique lié au groupe amino, ayant une activité antiobésité et modulatrice de la motricité intestinale.

On a maintenant trouvé que les analogues de phényléthanolamines décrits dans les demandes de brevets ci-dessus peuvent être utilisés pour le traitement des affections oculaires, notamment de l'hypertension oculaire et du glaucome.

Ainsi, la présente invention concerne, selon un de ses aspects, l'utilisation d'un analogue de phényléthanolamine ou d'un de ses sels pharmaceutiquement acceptables pour la préparation de compositions pharmaceutiques destinées au traitement des affections oculaires, notamment au contrôle de la pression intraoculaire et au traitement de l'hypertension oculaire et du glaucoma, ledit analogue de phényléthanolamine ayant la formule 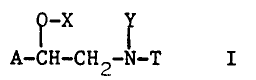 dans laquelle:
- A représente un groupe benzofuran-2-yle ou un groupe phényle, non substitués ou substitués par un ou deux atomes d'halogène ou par un groupe alkyle inférieur ou trifluorométhyle;
- X représente l'hydrogène, un alkyle inférieur ou un alcanoyle inférieur;
- Y représente l'hydrogène ou un groupe A′-CH(OH)-CH₂-, A′ étant identique à A, mais autre que benzofuran-2-yle; ou bien
- X et Y, pris ensemble, forment un groupe méthylène, éventuellement substitué par un groupe carbalcoxy inférieur; un groupe éthylène, éventuellement substitué par un groupe oxo; ou un groupe 1,3-propylène;
- T représente un groupe de formule II 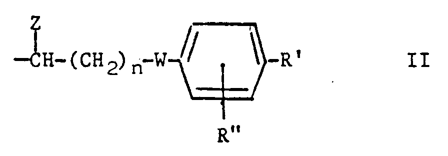 dans laquelle
   . n est 1, 2 ou 3;
   . Z représente l'hydrogène ou un alkyle inférieur;
   . W représente une liaison directe ou un atome d'oxygène;
   . R′ représente
      ° l'hydrogène;
      ° un groupe alkyle inférieur;
      ° un groupe fonctionnel choisi parmi les groupements suivants: hydroxy; alcoxy inférieur; alcényloxy inférieur; alcynyloxy inférieur; cycloalkyloxy; cycloalkyl-alcoxy inférieur; benzyloxy; phénoxy; mercapto; (alkyle inférieur)thio; (alcényle inférieur)thio; (alcynyle inférieur)thio; cycloalkylthio; (cycloalkyl-alkyle inférieur)thio; benzylthio; phénylthio; (alkyle inférieur)sulfinyle; (alcényle inférieur)sulfinyle; (alcynyle inférieur)sulfinyle; cycloalkylsulfinyle; (cycloalkyl-alkyle inférieur)sulfinyle; benzylsulfinyle; phénylsulfinyle; (alkyle inférieur)sulfonyle; (alcényle inférieur) sulfonyle; (alcynyle inférieur)sulfonyle; cycloalkylsulfonyle; (cycloalkyl-alkyle inférieur) sulfonyle; benzylsulfonyle; phénylsulfonyle; cyano; nitro; amino non substitué ou substitué par un ou deux radicaux, identiques ou différents, choisis parmi alkyle inférieur, alcényle inférieur, alcynyle inférieur, cycloalkyle, cycloalkyl-alkyle inférieur, benzyle, phényle; carboxy; carbalcoxy inférieur; (alcényloxy inférieur)carbonyle; (alcynyloxy inférieur)carbonyle; cycloalkyloxycarbonyle; (cycloalkyl-alcoxy inférieur)carbonyle; benzyloxycarbonyle; phénoxycarbonyle; carbamoyle non substitué ou substitué sur le groupe amino par un ou deux radicaux, identiques ou différents, choisis parmi les groupes alkyle inférieur, alcényle inférieur, alcynyle inférieur, cycloalkyle, cycloalkyl-alkyle inférieur, benzyle, phényle;
      ° un radical R choisi parmi les groupes alkyle inférieur substitués par un groupe fonctionnel, les groupes alcényle inférieur substitués par un groupe fonctionnel, les groupes alcynyle inférieur substitués par un groupe fonctionnel, les groupes phényl(alkyle inférieur) substitués sur le groupe phényle par un alkyle inférieur ou par un groupe fonctionnel, les groupes phényl(alcényle inférieur) substitués sur le groupe phényle par un alkyle inférieur ou par un groupe fonctionnel, les groupes phényl(alcynyle inférieur) substitués sur le groupe phényle par un alkyle inférieur ou par un groupe fonctionnel, les groupes benzyle substitués sur le groupe phényle par un alkyle inférieur ou par un groupe fonctionnel, ou les groupes phényle non substitués ou substitués par un alkyle inférieur ou par un groupe fonctionnel, le groupe fonctionnel étant comme défini ci-dessus;
      ° un groupe O-R, S-R , SO-R ou SO₂R, dans lesquels R est tel que défini ci-dessus;
      ° un groupe NRR°, où R est comme défini ci-dessus et R° représente l'hydrogène ou est tel que défini ci-dessus pour R, ou bien R et R° forment, avec l'azote auquel ils sont liés, un groupe choisi parmi les groupes pyrrolidino, pipéridino et morpholino;
      ° un groupe COOR ou un groupe CO-SR, dans lesquels R est tel que défini ci-dessus;
      ° un groupe CONRR°, où R est comme défini ci-dessus et R° représente l'hydrogène ou est tel que défini ci-dessus pour R, ou bien R et R° forment, avec l'azote auquel ils sont liés, un groupe choisi parmi les groupes pyrrolidino, pipéridino et morpholino;
      ° un groupe SO₂NRR°, où R est tel que défini ci-dessus et R° représente l'hydrogène ou est comme défini ci-dessus pour R, ou bien R et R° forment, avec l'azote auquel ils sont liés, un groupe choisi parmi les groupes pyrrolidino, pipéridino et morpholino; et
   . R˝ représente
      ° l'hydrogène;
      ° un halogène;
      ° un alkyle inférieur;
      ° un groupe fonctionnel tel que défini ci-dessus;
      ° un groupe O-R, R étant tel que défini ci-dessus;
      ° un groupe COOR, R étant tel que défini ci-dessus;
      ° un groupe CONRR°, où R est tel que défini ci-dessus et R° représente l'hydrogène ou est comme défini ci-dessus pour R, bien R et R° forment, avec l'azote auquel ils sont liés, un groupe choisi parmi les groupes pyrrolidino, pipéridino et morpholino;
   ou bien,
- T peut aussi représenter, lorsque A représente un groupe phényle non substitué ou substitué par un atome d'halogène, un groupe alkyle inférieur ou un groupe trifluorométhyle, et X et Y représentent l'hydrogene, un groupe de formule III 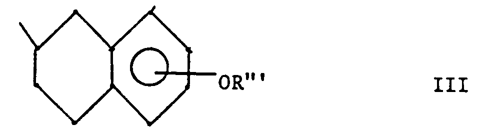 dans laquelle -R‴ représente l'hydrogène; un groupe méthyle non substitué ou substitué par un groupe carboxy ou carbalcoxy inférieur; ou un groupe alkyle de 3 à 5 atomes de carbone à chaîne droîte ou ramifiée substitué par un groupe carboxy ou carbalcoxy inférieur.

Les termes "alkyle inférieur", "alcényle inférieur" et "alcynyle inférieur", tels qu'utilisés ici, désignent les radicaux monovalents dérivés d'hydrocarbures aliphatiques saturés ou contenant une double ou une triple liaison et ayant jusqu'à 4 atomes de carbone, tel que par exemple les groupes méthyle, éthyle, propyle, isopropyle, t-butyle, allyle, crotyle, propargyle.

Le terme "cycloalkyle" désigne le radical monovalent d'un hydrocarbure alicyclique contenant de 3 à 8 atomes de carbone, le cyclopropyle, le cyclopentyle et le cyclohexyle étant particulièrement préférés.

Les termes "alcoxy inférieur", "alcényloxy inférieur", "alcynyloxy inférieur", "cycloalkyloxy" et les dérivés soufrés correspondants, tels qu'utilisés ici, désignent le groupe hydroxyle ou thiol éthérifié par un alkyle inférieur, par un alcényle inférieur, par un alcynyle inférieur ou par un cycloalkyle, tels que définis ci-dessus.

Le terme "carbalcoxy inférieur" désigne un ester d'alkyle inférieur du groupe carboxyle, le terme alkyle inférieur étant tel que défini ci-dessus; plus particulièrement, les termes "carbalcoxy", "carbométhoxy" et "carbéthoxy" sont utilisés ici comme synonymes des termes alkoxycarbonyle, méthoxycarbonyle et éthoxycarbonyle.

Le terme "alcanoyle inférieur" désigne le groupe carbonyle substitué par un alkyle inférieur tel que défini ci-dessus.

Le terme "halogène" désigne les quatre halogènes courants, à savoir le fluor, le chlore, le brome et l'iode, le fluor et, plus particulièrement, le chlore étant préférés.

Les composés de formule I ci-dessus peuvent être sous une forme optiquement inactive ou sous une forme optiquement active choisie parmi celles des énantiomères, des diastéréoisomères et de leurs mélanges. Tous ces composés et leurs sels pharmaceutiquement acceptables peuvent être utilisés dans le cadre de la présente invention.

Les sels pharmaceutiquement acceptables des composés de formule I, par exemple le chlorhydrate, le bromhydrate, le sulfate, l'hydrogénosulfate, le dihydrogénophosphate, le méthanesulfonate, le méthylsulfate, l'oxalate, le maléate, le fumarate, le naphtalène-2-sulfonate, sont ceux qui sont obtenus par traitement de la base libre, dissoute par exemple dans un alcool tel que l'isopropanol, avec une solution de l'acide choisi dans le même solvant.

Lorsque le composé de formule I possède un groupe carboxyle libre, son caractère amphotère permet la préparation de sels soit avec des acides soit avec des bases pharmaceutiquement acceptables. Les sels avec des bases pharmaceutiquement acceptables sont de préférence ceux avec des métaux alcalins tels que le sodium, mais les sels avec des bases organiques, tels que le sel avec le trométamol, sont également convenables.

Sont préférés les composés de formule I ci-dessus, dans laquelle A représente un groupe phényle non substitué ou substitué par un atome d'halogène, un groupe alkyle inférieur, ou un groupe trifluorométhyle, X et Y sont l'hydrogène et T représente un groupe de formule III dans laquelle R‴ est tel que défini ci-dessus.

Encore plus préférés sont les composés de formule (I) dans laquelle A est un groupe 3-chlorophényle, X et Y sont l'hydrogène et T est un groupe de formule III dans laquelle -R‴ est l'hydrogène ou un groupe carbométhoxyméthyle, carbéthoxyméthyle, carbométhoxybutyle, carboéthoxybutyle, 2-carbométhoxy-2-propyle, ou 2-carboéthoxy-2-propyle.

Des principes actifs particulièrement avantageux sont
- le 2-[(7-hydroxy-1,2,3,4-tétrahydronapht-2-yl)amino]-1-(3-chlorophényl)éthanol ("COMPOSE A") et ses sels pharmaceutiquement acceptables (EP 211721);
- la N-[(2S)-7-hydroxy-1,2,3,4-tétrahydronapht-2-yl]-(2R)-2-(3-chlorophényl)-2-hydroxyéthanamine ("COMPOSE B") et ses sels pharmaceutiquement acceptables (EP 303545);
- la N-[(2R)-7-hydroxy-1,2,3,4-tétrahydronapht-2-yl]-(2R)-2-(3-chlorophényl)-2-hydroxyéthanamine ("COMPOSE C") et ses sels pharmaceutiquement acceptables (EP 303545);
- la N-[(2S)-7-hydroxy-1,2,3,4-tétrahydronapht-2-yl]-(2S)-2-(3-chlorophényl)-2-hydroxyéthanamine ("COMPOSE D") et ses sels pharmaceutiquement acceptables (EP 303545);
- la N-[(2R)-7-hydroxy-1,2,3,4-tétrahydronapht-2-yl]-(2S)-2-(3-chlorophényl)-2-hydroxyéthanamine ("COMPOSE E") et ses sels pharmaceutiquement acceptables (EP 303545);
- la N-[(2R)-7-éthoxycarbonylméthoxy-1,2,3,4-tétrahydronapht-2-yl]-(2R)-2-(3-chlorophényl)-2-hydroxyéthanamine ("COMPOSE F") et ses sels pharmaceutiquement acceptables (EP 303546);
- la N-[(2S)-7-éthoxycarbonylméthoxy-1,2,3,4-tétrahydronapht-2-yl]-(2R)-2-(3-chlorophényl)-2-hydroxyéthanamine ("COMPOSE G") et ses sels pharmaceutiquement acceptables (EP 303546);
- la N-[(2S)7-éthoxycarbonylméthoxy-1,2,3,4-tétrahydronapht-2-yl]-(2S)-2-(3-chlorophényl)-2-hydroxyéthanamine ("COMPOSE H") et ses sels pharmaceutiquement acceptables (EP 303546);
- la N-[(2R)-7-(méthoxycarbonylmethoxy)--1,2,3,4-tétrahydronapht-2-yl]-(2R)-(3-chlorophényl)-2-hydroxyéthanamine ("COMPOSE I") et ses sels pharmaceutiquement acceptables (EP 303546);
- la N-[7-(éthoxycarbonylbutan-4-yloxy)--1,2,3,4-tétrahydronapht-2-yl]-2-(3-chlorophenyl)-2-hydroxyéthanamine ("COMPOSE J") et ses sels pharmaceutiquement acceptables (demande de brevet européen n. 90 400 405.8) ;
- la N-[7-(2-éthoxycarbonylpropan-2-yloxy)-1,2,3,4-tétrahydronapht-2-yl]-2-(3-chlorophenyl)-2-hydroxyéthanamine ("COMPOSE K") et ses sels pharmaceutiquement acceptables (demande de brevet européen n 90 400 405.8).

Sont aussi préférés les composés de formule I ci-dessus dans laquelle
- A est un groupe phényle, 2-fluorophényle, 3-chlorophényle ou 3-trifluorométhylphényle;
- X est l'hydrogène;
- Y est l'hydrogène ou un groupe A′-CH(OH)-CH₂-, où A′ est un groupe phényle, 2-fluorophényle, 3-chlorophényle ou 3-trifluorométhylphényle identique à A;
- T est un groupe de formule II ci-dessus dans laquelle
   . n est 1 ou 2;
   . R′ est
      ° un groupe fonctionnel choisi parmi les groupes hydroxy, carbalcoxy inférieur, carboxy, carbamoyle; ou
      ° un groupe O-R, R étant ou un groupe alkyle inférieur ou alcényle inférieur substitué par un groupe fonctionnel choisi parmi les groupes carbalcoxy inférieur et carboxy;
   . R˝ est l'hydrogène;
   . W est une liaison directe; et
   . Z est un alkyle inférieur, notamment méthyle.

Plus encore préférés pour l'utilisation selon la présente invention sont les phényléthanolamines représentés par la formule I ci-dessus, dans laquelle
- A est un groupe phényle, 3-chlorophényle ou 3-trifluorométhylphényle;
- Y est l'hydrogène ou un groupe A′-CH(OH)-CH₂- où A′, identique à A, est phényle;
- X est l'hydrogène,
- T représente un groupe de formule II ci-dessus dans laquelle
   . n est 1 ou 2;
   . R′ est un groupe hydroxy, carbométhoxy, carbéthoxy, carboxy, carbamoyle, carboxyméthoxy, carbométhoxyméthoxy ou carbéthoxyméthoxy;
   . R˝ est l'hydrogène;
   . W est une liaison directe; et
   . Z est méthyle.

Parmi ces composés, des principes actifs particulièrement avantageux sont
- la N-[2-carbométhoxyphényl)-1-méthyléthyl]-2-hydroxy-2-phényléthylamine ci-après désignée "COMPOSE L", décrite dans l'Exemple 21 du brevet européen 6735, sous forme de mélange de diastéréoisomères de plus bas point de fusion préparé comme illustré dans la "Description 15" du brevet européen 21636; et ses sels pharmaceutiquement acceptables, notamment le fumarate neutre;
- la N-[2-(4-carboxyphényl)-1-méthyléthyl]-2-hydroxy-2-phényléthylamine, ci-après désignée "COMPOSE M", préparée par saponification du Composé L, et ses sels pharmaceutiquement acceptables;
- la (R,S)-N-(2-phényl-2-hydroxyéthyl)-1-méthyl-3-(4-hydroxyphényl)propylamine, ci-après désignée "COMPOSE N", décrite dans l'Exemple 10 du brevet USA 4,391,826; et ses sels pharmaceutiquement acceptables, notamment le chlorhydrate;
- la (R,S)-N-(2-phényl-2-hydroxyéthyl)-1-méthyl-3-(4-aminocarbonylphényl)propylamine, ci-après désignée "COMPOSE O", décrite dans l'Exemple 15 du brevet USA 4,391,826; et ses sels pharmaceutiquement acceptables, notamment le chlorhydrate;
- le p-[(R)-3-[bis-[(R)-béta-hydroxyphénéthyl]amino]butyl]benzamide, ci-après désigné "COMPOSE P", décrit dans l'Exemple 12 de la demande de brevet européen 101069, et ses sels pharmaceutiquement acceptables, notamment le maléate et le fumarate;
- le p-[(S)-3-[bis-[(R)-béta-hydroxyphénéthyl]amino]butyl]benzamide, ci-après désigné "COMPOSE Q", décrit dans l'Exemple 8 de la demande de brevet européen 101069; et ses sels pharmaceutiquement acceptables;
- la (RR,SS)-N-[2-(4-carbométhoxyphényl)-1-méthyléthyl]-2-hydroxy-2-(3-trifluorométhylphényl)éthanamine, ci-après désignée "COMPOSE R", décrite dans l'Exemple 19 du brevet européen 40915, et ses sels pharmaceutiquement acceptables, notamment le chlorhydrate;
- la (RR,SS)-N-[2-(4-carboxyphényl)-1-méthyléthyl]-2-hydroxy-2-(3-trifluorométhylphényl)éthanamine, ci-après désignée "COMPOSE S", préparée par saponification du Composé A; et ses sels pharmaceutiquement acceptables;
- la N-[2-(4-carbométhoxyméthoxyphényl)-1-méthyléthyl]-2-hydroxy-2-(3-chlorophényl)éthanamine, ci-après désignée "COMPOSE T", décrite dans l'Exemple 6 du brevet européen 23385, et ses sels pharmaceutiquement acceptables, notamment le bromhydrate de sa forme (RR,SS) ("Description 21" du brevet européen 70133);
- la N-[2-(4-carboxyméthoxyphényl)-1-méthyléthyl]-2-hydroxy-2-(3-chlorophényl)éthanamine, ci-après désignée "COMPOSE U", préparée par saponification du Composé T; et ses sels pharmaceutiquement acceptables.

Les composés de formule I ci-dessus peuvent être préparés comme décrit dans les demandes de brevets européens publiées sous les numéros 6735, 7204, 21636, 23385, 25331, 28105, 40000, 40915, 52963, 61907, 63004, 66351, 68669, 70133, 70134, 89154, 91749, 95827, 99707, 101069, 140359, 146392, 164700, 170121, 170135, 171519, 211721, 255415, 262785, 303545, 303546 dans le brevet belge 900.983, dans la demande de brevet anglais 2133986, dans la demande de brevet australien 84/31944, dans la demande de brevet internationale WO 84/00956, dans le brevet USA 4,391,826, et dans la demande de brevet européen n. 90 400 405.8.

Les composés de formule I et leurs sels pharmaceutiquement acceptables sont peu toxiques. Leur toxicité aigue est bien inférieure à la dose prévue pour le traitement thérapeutique.Ils sont bien tolérés localement après administration oculaire.

En particulier, certains composés de formule I ont été indiqués comme agents thérapeutiques potentiels dans le traitement de l'obésité (Nature, 1984, 309, 163-165) et un d'entre eux a été essayé en clinique humaine (Int. J. Obesity, 1985, 9, 230; ibid. 1985, 9, 231).

La présente invention concerne également, selon un autre de ses aspects, une méthode pour le traitement des affections oculaires, notamment pour le contrôle de la pression intraoculaire et le traitement de l'hypertension oculaire et du glaucome chez les mammifères, y compris l'homme, ladite méthode étant caractérisée en ce qu'on administre aux dits mammifères une quantité efficace d'un composé de formule I ou d'un de ses sels pharmaceutiquement acceptables.

L'invention se rapporte également à l'utilisation d'un analogue de phényléthanolamine de formule I ou d'un de ses sels pharmaceutiquement acceptables en combinaison avec un autre principe actif indiqué pour le traitement du glaucome. Le principe actif utilisé en combinaison avec le composé de l'invention peut être un antiinflammatoire notamment un antiinflammatoire stéroidien ou corticostéroide pour le traitement du glaucome dont un effet secondaire est l'augmentation de la pression intraoculaire.

La présente invention concerne également une composition pharmaceutique contenant un analogue de phényléthanolamine de formule I ou un de ses sels pharmaceutiquement acceptables et destinée au traitement de l'augmentation de la pression intraoculaire consécutive à un traitement par des antiinflammatoires stéroidiens.

Les analogues de phényléthanolamine utilisés selon la présente invention sont administrés de préférence sous forme de compositions pharmaceutiques ophtalmiques pour une administration topique sur l'oeil, comme des solutions, des suspensions ou des pommades.

Les formulations ophtalmiques selon l'invention peuvent contenir de 0,00001 à 1 % en poids d'un composé de la présente invention, plus particulièrement de 0,0001 à 0,2 %. Chaque unité de dosage (goutte) comprend une quantité de phényléthanolamine comprise entre 10 ng et 1 mg, préférentiellement entre 100 ng à 0,2 mg.

L'administration de ces formulations peut être effectuée par application dans l'oeil de 1-2 gouttes 1 à 3 fois par jour de façon à assurer une posologie journalière de 10 ng à 1 mg, de préférence de 100 ng à 0,2 mg de principe actif.

L'expression "contrôle de la pression intraoculaire élevée", telle qu'utilisée dans la présente description, signifie la régulation, la diminution et la modulation de la tension intraoculaire élevée qui est le premier symptôme permettant le diagnostic du glaucome. L'expression signifie également que la diminution de la pression intraoculaire obtenue selon l'invention par utilisation d'un analogue de phényléthanolamine de formule I ou d'un de ses sels est maintenue pour une période de temps suffisante, par exemple entre l'administration de deux doses consécutives.

L'effet d'abaissement de la pression intraoculaire d'une composition pharmaceutique selon l'invention peut être déterminé chez l'animal, par exemple chez le lapin, dans un test qui prévoit l'administration orale de quantités importantes d'eau, tel que décrit par exemple dans Arch. Ophthal., 1969, 82, 381-384 ou dans J. Ocul. Pharmacol, 1985,1 (2), 161-168, ou l'injection rapide par voie intraveineuse d'une solution de glucose, tel que décrit par exemple dans Boll. Ocul., 1979, 58(7-8), 359-366.

Pour préparer des formulations convenables, les compositions pharmaceutiques peuvent être mélangées avec un véhicule convenable soit pour une administration ophtalmique topique, soit pour une administration par voie générale. Comme véhicules pharmaceutiques acceptables pour une administration ophtalmique, on peut citer l'eau, un mélange d'eau et de solvants miscibles à l'eau comme les alcanols inférieurs, les huiles végétales, les huiles minérales et comprenant de 0,5 à 5 % en poids d'hydroxyéthylcellulose, d'oléate d'éthyle, de carboxyméthylcellulose, de polyvinylpyrrolidone et d'autres polymères solubles dans l'eau non toxiques et compatibles avec une utilisation ophtalmique, par exemple des dérivés de la cellulose tels que le méthylcellulose, un dérivé d'un métal alcalin de carboxyméthylcellulose, l'hydroxyméthylcellulose, l'hydroxyéthylcellulose, l'hydroxypropylméthylcellulose, des acrylates tels que des sels d'acides polyacryliques, des éthylcrylates, des polycrylamides, des produits naturels tels que la gélatine, les alginates, les pectines, le tragacanthe, le karaya, le chondrus, l'agar, l'acacia, des dérivés d'amidon comme l'acétate d'amidon, les éthers d'hydroxyéthylamidon, l'hydroxypropylamidon, ainsi que d'autres dérivés synthétiques comme le polyvinylalcool, le polyvinylpyrrolidone, le polyvinylméthyléther, le polyéthylèneoxyde, le carbopol neutre ou le xanthane ou des mélanges de ces polymères. La préparation pharmaceutique peut aussi contenir des substances auxiliaires non toxiques comme des émulsifiants, des conservateurs, des agents de mouillage, des agents texturant et d'autres comme par exemple les polyéthylènes glycols 200, 300, 400, 600, les Carbowax® 1000, 1500, 4000, 6000, 10000, des produits antibactériens, comme des ammonium quarternaires, des sels phénylmercuriques connus pour avoir des propriétés stérilisantes à froid sans être agressifs, le timérosal, le propylparaben, l'alcool benzylique, le phényl éthanol, des agents tampons comme un chlorure de métal alcalin, des tampons borate, acétate ou gluconate, des antioxydants comme le métabisulfite de sodium, l'hydroxyanisol butylé, l'hydroxytoluène butylé ou des agents semblables et d'autres agents classiquement utilisés comme le monolaurate de sorbitan, l'oléate de triéthanolamine, le monopalmitate de polyéthylène sorbitan, un sel de métal alcalin du dioctyl sulfosuccinate, le monothioglycérol, l'acide éthylènediamine tétracétique ou autres.

De plus, des excipients ophtalmiques acceptables peuvent être utilisés comme le tampon phosphate, l'acide borique isotonique, un chlorure alcalin isotonique, ou la trométhamine par exemple.

La préparation pharmaceutique peut également être une suspension dans laquelle les particules solubles sont des polymères solubles dans l'eau ou insolubles. Une telle suspension peut contenir des microformes telles que des microparticules ou des nanoparticules.

Les analogues de phényléthanolamine utilisés selon la présente invention peuvent également être administrés sous forme de compositions pharmaceutiques pour une administration entérale ou parentérale; de telles compositions et dosages sont décrits dans EP 211721, EP 255415 et dans la demande de brevet européen 90400 405.8.

Les compositions selon l'invention peuvent contenir des agents thérapeutiques additionnels. Ainsi des antibiotiques, des anesthésiques ou d'autres agents abaissant la pression intraoculaire peuvent être présents.

### EXEMPLE 1.

### Solution ophtalmique:

| | |
|---|---|
| Composé G (comme chlorhydrate) | 1,0 mg |
| NaH₂PO₄ | 10,4 mg |
| Na₂HPO₄ | 2,4 mg |
| chlorobutanol | 5,0 mg |
| hydroxypropylméthylcellulose | 5,0 mg |
| NaOH 1N q.s. | pH = 7,4 |
| eau distillée q.s.p. | 1,0 ml |

Les constituants de la solution sont mélangés dans les conditions habituelles pour obtenir une solution ophtalmique.

### EXEMPLE 2.

### Solution ophtalmique:

| | |
|---|---|
| Composé F (comme chlorhydrate) | 1,0 mg |
| NaH₂PO₄ | 10,4 mg |
| Na₂HPO₄ | 2,4 mg |
| chlorobutanol | 5,0 mg |
| hydroxypropylméthylcellulose | 5,0 mg |
| NaOH 1N q.s. | pH = 7,4 |
| eau distillée q.s.p. | 1,0 ml |

Les constituants de la solution sont mélangés dans les conditions habituelles pour obtenir une solution ophtalmique.

### EXEMPLE 3.

### Solution ophtalmique:

| | |
|---|---|
| Composé K (comme chlorhydrate) | 1,0 mg |
| phosphate sodique de bétamétasone | 1,0 mg |
| NaH₂PO₄ | 10,4 mg |
| Na₂HPO₄ | 2,4 mg |
| chlorbutanol | 5,0 mg |
| hydroxypropylméthylcellulose | 5,0 mg |
| NaOH 1N q.s. | pH = 7,4 |
| eau distillée q.s.p. | 1,0 ml |

Les constituants de la solution sont mélangés dans les conditions habituelles pour obtenir une solution ophtalmique.

### EXEMPLE 4.

### Solution ophtalmique :

| | |
|---|---|
| Composé L (comme fumarate neutre) | 1,0 mg |
| NaH₂PO₄ | 10,4 mg |
| Na₂HPO₄ | 2,4 mg |
| chlorobutanol | 5,0 mg |
| hydroxypropylméthylcellulose | 5,0 mg |
| NaOH 1N q.s. | pH = 7,4 |
| eau distillée q.s.p. | 1,0 ml |

Les constituants de la solution sont mélangés dans les conditions habituelles pour obtenir une solution ophtalmique.

### EXEMPLE 5.

### Solution ophtalmique:

| | |
|---|---|
| Composé U (comme bromhydrate) | 1,0 mg |
| NaH₂PO₄ | 10,4 mg |
| Na₂HPO₄ | 2,4 mg |
| chlorobutanol | 5,0 mg |
| hydroxypropylméthylcellulose | 5,0 mg |
| NaOH 1N q.s. | pH = 7,4 |
| eau distillée q.s.p. | 1,0 ml |

Les constituants de la solution sont mélangés dans les conditions habituelles pour obtenir une solution ophtalmique.

### EXEMPLE 6.

### Solution ophtalmique:

| | |
|---|---|
| Composé U | 1,0 mg |
| phosphate sodique de bétamétasone | 1,0 mg |
| NaH₂PO₄ | 10,4 mg |
| Na₂HPO₄ | 2,4 mg |
| chlorbutanol | 5,0 mg |
| hydroxypropylméthylcellulose | 5,0 mg |
| NaOH 1N q.s. | pH = 7,4 |
| eau distillée q.s.p. | 1,0 ml |

Les constituants de la solution sont mélangés dans les conditions habituelles pour obtenir une solution ophtalmique.

### EXEMPLE 7.

### Evaluation pharmacologique

On évalue l'activité pharmacologique des composés de formule I selon la présente invention sur la pression intraoculaire chez le lapin hypertendu en utilisant le modèle de glaucome expérimental mis au point par L. Bonomi (1976). Dans ce modèle on provoque l'élévation immédiate de la pression intraoculaire (PIO) du lapin par l'injection rapide, par voie intraveineuse, de 15 mg/kg d'une solution de glucose à 5%. La PIO atteint son maximum (29 ± 1,6 mmHg) en 5 minutes puis retrouve son niveau presque normal (22 ±1,6 mmHg) en 40 minutes. Dans ces tests on utilise des lapins pigmentés, Fauve de Bourgogne, femelles (3-4 kg) ayant une pression intra-oculaire de base (PIO base) normale dans les deux yeux (19 ± 0,6 mmHg). Une dose unique de 50 ul de collyre (un des produits à tester dans une solution physiologique ou le seul vehicule) a été instillée, dans un oeil, en aveugle (l'oeil opposé étant le temoin non traité). 10 minutes après cette instillation, une deuxième mesure de PIO a été réalisée.
Immédiatement après cette mesure, 15 mg/kg d'une solution de glucose à 5% ont été injectés rapidement dans la veine marginale de l'oreille. La PIO a été alors mesurée 5 mn, 10 mn, 20 mn, 30 mn, et 40 mn après la fin de l'injection. Les mesures ont été effectuées à l'aide d'un tonomètre pnéumatique.
Lorsque l'instillation du vehicule n'a produit aucune différence significative de la courbe d'elévation de la PIO par rapport à celle des yeux non traités, les résultats obtenus avec des composés représentatives de la présente invention montrent que les composés de formule I sont actifs sur ce modèle de glaucome aigu.
A titre d'exemple l'instillation du composé G (comme chlorhydrate), ne provoque pas de diminution de la PIO de base 10 minutes après son instillation, tandis qu'il permet de maintenir la valeur maximale de PIO atteinte en 5 minutes au-dessous de 21 ± 2,3 mmHg et de reporter la PIO à sa valeur normale très rapidement.

## Revendications

1. Utilisation d'un analogue de phényléthanolamine ou d'un de ses sels pharmaceutiquement acceptables pour la préparation de compositions pharmaceutiques destinées au traitement des affections oculaires, ledit analogue de phényléthanolamine ayant la formule 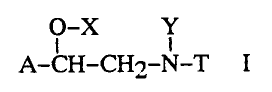 dans laquelle :
- A représente un groupe benzofuran-2-yle ou un groupe phényle, non substitués ou substitués par un ou deux atomes d'halogène ou par un groupe (C₁-C₄) alkyle ou trifluorométhyle;
- X représente l'hydrogène, un (C₁-C₄)alkyle ou un (C₁-C₄)alcanoyle ;
- Y représente l'hydrogène ou un groupe A'-CH(OH)-CH₂-, A' étant identique à A, mais autre que benzofuran-2-yle ; ou bien
- X et Y, pris ensemble, forment un groupe méthylène, éventuellement substitué par un groupe carbalcoxy dans lequel le groupe alcoxy est en (C₁-C₄) ; un groupe éthylène, eventuellement substitué par un groupe oxo ; ou un groupe 1,3-propylène ;
- T représente un groupe de formule II 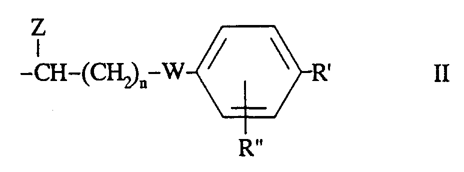 dans laquelle
. n est 1, 2 ou 3;
. Z représente l'hydrogène ou un (C₁-C₄)alkyle ;
. W représente une liaison directe ou un atome d'oxygène ;
. R' représente
• l'hydrogène ;
• un groupe (C₁-C₄) alkyle;
• un groupe fonctionnel choisi parmi les groupements suivants : hydroxy ; (C₁-C₄) alcoxy; (C₂-C₄)alcényloxy ; (C₂-C₄)alcynyloxy ; cycloalkyloxy ; cycloalkyl-(C₁-C₄)alcoxy ; benzyloxy ; phénoxy ; mercapto ; ((C₁-C₄)alkyle)thio ; ((C₂C₄)alcényle)thio ; ((C₂-C₄)alcynyle)thio ; cycloalkylthio ; (cycloalkyl-(C₁-C₄)alkyle)thio ; benzylthio ; phénylthio; ((C₁-C₄)alkyle)sulfinyle ; ((C₂-C₄)alcényle)sulfinyle ; ((C₂-C₄)alcynyle)-sulfinyle ; cycloalkysulfinyle ; (cycloalkyl-(C₁-C₄)alkyle)sulfinyle ; benzylsulfinyle ; phénylsulfinyle ; ((C₁-C₄)alkyle)sulfonyle ; ((C₂-C₄)alcényle)sulfonyle ; ((C₂-C₄)alcynyle)sulfonyle ; cycloalkylsulfonyle ; (cycloalkyl-(C₁-C₄)alkyle)sulfonyle ; benzylsulfonyle ; phénylsulfonyle ; cyano ; nitro ; amino non substitué ou substitué par un ou deux radicaux, identiques ou différents, choisis parmi (C₁-C₄)alkyle, (C₂-C₄)alcényle, (C₂-C₄)alcynyle, cycloalkyle, cycloalkyl-(C₁-C₄) alkyle, benzyle, phényle ; carboxy ; carbalcoxy dans lequel le groupe alcoxy est en C₁-C₄ ; ((C₂-C₄)alcényloxy)carbonyle ; ((C₂-C₄)alcynyloxy)carbonyle ; cycloalkyloxycarbonyle ; (cycloalkyl-(C₁-C₄)alcoxy)carbonyle ; benzyloxycarbonyle ; phénoxycarbonyle ; carbamoyle non substitué ou substitué sur le groupe amino par un ou deux radicaux, identiques ou différents, choisis parmi les groupes (C₁-C₄)alkyle, (C₂-C₄)alcényle, (C₂-C₄)alcynyle, cycloalkyle, cycloalky-(C₁-C₄)alkyle, benzyle, phényle ;
• un radical R choisi parmi les groupes (C₁-C₄)alkyle substitués par un groupe fonctionnel, les groupes (C₂-C₄)alcényle substitués par un groupe fonctionnel, les groupes (C₂-C₄)alcynyle substitués par un groupe fonctionnel, les groupes phényl((C₁-C₄)alkyle) substitués sur le groupe phényle par un (C₁-C₄)alkyle ou par un groupe fonctionnel, les groupes phényl((C₂-C₄)alcényle) substitués sur le groupe phényle par un (C₁-C₄)alkyle ou par un groupe fonctionnel, les groupes phényl((C₂-C₄)alcynyle) substitués sur le groupe phényle par un (C₁-C₄)alkyle ou par un groupe fonctionnel, les groupes benzyle substitués sur le groupe phényle par un (C₁-C₄)alkyle ou par un groupe fonctionnel, ou les groupes phényle non substitués ou substitués par un (C₁-C₄)alkyle ou par un groupe fonctionnel, le groupe fonctionnel étant comme défini ci-dessus ;
• un groupe O-R, S-R, SO-R ou SO₂R, dans lequel R est tel que défini ci-dessus ;
• un groupe NRR^{·}, où R est comme défini ci-dessus et R^{·} représente l'hydrogène ou est tel que défini ci-dessus pour R, ou bien R et R^{·} forment, avec l'azote auquel ils sont liés, un groupe choisi parmi les groupes pyrrolidino, pipéridino et morpholino ;
• un groupe COOR, ou un groupe CO-SR, dans lequel R est tel que défini ci-dessus ;
• un groupe CONRR^{·}, où R est comme défini ci-dessus et R^{·} représente l'hydrogène ou est tel que défini ci-dessus pour R, ou bien R et R^{·} forment, avec l'azote auquel ils sont liés, un groupe choisi parmi les groupes pyrrolidino, pipéridino et morpholino ; et
• un groupe SO₂NRR^{·}, où R est tel que défini ci-dessus et R^{·} représente l'hydrogène ou est comme défini ci-dessus pour R, ou bien R et R^{·} forment, avec l'note auquel ils sont liés, un groupe choisi parmi les groupes pyrrolidino, pipéridino et morpholino ; et
. R'' représente
• l'hydrogène ;
• un halogène ;
• un (C₁-C₄)alkyle ;
• un groupe fonctionnel tel que défini ci-dessus ;
• un groupe O-R, R étant tel que défini ci-dessus ;
• un groupe COOR, R étant tel que défini ci-dessus ;
• un groupe CONRR^{·}, où R est tel que défini ci-dessus et R^{·} représente l'hydrogène ou est comme défini ci-dessus pour R, ou bien R et R^{·} forment, avec l'azote auquel ils sont liés, un groupe choisi parmi les groupes pyrrolidino, pipéridino et morpholino ; ou bien,
- T peut aussi représenter, lorsque A représente un groupe phényle non substitué ou substitué par un atome d'halogène, un groupe (C₁-C₄)alkyle ou un groupe trifluorométhyle, et X et Y représentent l'hydrogène, un groupe de formule III 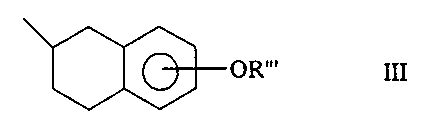 dans laquelle -R''' représente l'hydrogène ou un groupe méthyle non substitué ou substitué par un groupe carboxy ou carbalcoxy dans lequel le groupe alcoxy en C₁-C₄ ; ou un groupe alkyle de 3 à 5 atomes de carbone à chaîne droite ou ramifiée substitué par un groupe carboxy ou carbalcoxy dans lequel le groupe alcoxy est en (C₁-C₄).

2. Utilisation selon la revendication 1, caractérisée en ce que l'analogue de phényléthanolamine a la formule I indiquée dans la revendication 1 dans laquelle
- A est un groupe phényle non substitué ou substitué par un atome d'halogène, un groupe (C₁-C₄)alkyle ou un groupe trifluorométhyle
- X et Y sont l'hydrogène, et
- T est un groupe de formule III dans laquelle R''' est tel que défini dans la revendication 1.

3. Utilisation selon la revendication 2, caractérisée en ce que l'analogue de phényléthanolamine a la formule I indiquée dans la revendication 1 dans laquelle
- A est un groupe phényle non substitué ou substitué par un atome d'halogène, un groupe (C₁-C₄)alkyle ou un groupe trifluorométhyle
- X et Y sont l'hydrogène, et
- T est un groupe de formule III dans laquelle R''' représente l'hydrogène ou un groupe méthyle non substitué ou substitué par un groupe carboxy ou carbalcoxy dans lequel le groupe alcoxy est en C₁-C₄.

4. Utilisation selon la revendication 2, caractérisée en ce que la phényléthanolamine a la formule I dans laquelle A est un groupe 3-chlorophényle et R''' est l'hydrogène, un groupe carbométhoxyméthyle, carboéthoxyméthyle, carbométhoxybutyle, carboéthoxybutyle, 2-carbométhoxy-2-propyle ou 2-carboéthoxy-2-propyle.

5. Utilisation selon la revendication 4, caractérisée en ce que la phényléthanolamine est choisie parmi le groupe qui consiste en :
- 2-[(7-hydroxy-1,2,3,4-tétrahydronapht-2-yl)amino]-1-(3-chlorophényl)éthanol et ses sels pharmaceutiquement acceptables ;
- N-[(2S)-7-hydroxy-1,2,3,4-tétrahydronapht-2-yl]-(2R)-2-(3-chlorophényl)-2-hydroxyéthanamine et ses sels pharmaceutiquement acceptables ;
- N-[(2R)-7-hydroxy-1,2,3,4-tétrahydronapht-2-yl]-(2R)-2-(3-chlorophényl)-2-hydroxyéthanamine et ses sels pharmaceutiquement acceptables ;
- N-[(2S)-7-hydroxy-1,2,3,4-tétrahydronapht-2-yl]-(2S)-2-(3-chlorophényl)-2-hydroxyéthanamine et ses sels pharmaceutiquement acceptables ;
- N-[(2R)-7-hydroxy-1,2,3,4-tétrahydronapht-2-yl]-(2S)-2-(3-chlorophényl)-2-hydroxyéthanamine et ses sels pharmaceutiquement acceptables ;
- N-[(2R)-7-éthoxycarbonylméthoxy-1,2,3,4-tétrahydronapht-2-yl]-(2R)-2-(3-chlorophényl)-2-hydroxyéthanamine et ses sels pharmaceutiquement acceptables ;
- N-[(2S)-7-éthoxycarbonylméthoxy-1,2,3,4-tétrahydronapht-2-yl]-(2R)-2-(3-chlorophényl)-2-hydroxyéthanamine et ses sels pharmaceutiquement acceptables ;
- N-[(2S)-7-éthoxycarbonylméthoxy-1,2,3,4-tétrahydronapht-2-yl]-(2S)-2(3-chlorophényl)-2-hydroxyéthanamine et ses sels pharmaceutiquement acceptables ; et
- N-[(2R)-7-méthoxycarbonylméthoxy-1,2,3,4-tétrahydronapht-2-yl]-(2R)-2-(3-chlorophényl)-2-hydroxyéthanamine et ses sels pharmaceutiquement acceptables ;
- la N-[7-(éthoxycarbonylbutan-4-yloxy)-1,2,3,4-tétrahydronapht-2-yl]-2-(3-chlorophényl)-2-hydroxyéthanamine et ses sels pharmaceutiquement acceptables ;
- la N-[7-(2-éthoxycarbonylpropan-2-yloxy)-1,2,3,4-tétrahydronapht-2-yl]-2-(3-chlorophényl)-2-hydroxyéthanamine et ses sels pharmaceutiquement acceptables.

6. Utilisation selon la revendication 1, caractérisée en ce que l'analogue de phényléthanolamine a la formule I indiquée dans la revendication 1 dans laquelle
- A est un groupe phényle, 2-fluorophényle, 3-chlorophényle ou 3-trifluorométhylphényle ;
- X est l'hydrogène ;
- Y est l'hydrogène ou un groupe A'-CH(OH)-CH₂-, où A' est un groupe phényle, 2-fluorophényle, 3-chlorophényle ou 3-trifluorométhylphényle identique à A ; et
- T est un groupe de formule II dans laquelle
. n est 1 ou 2 ;
. R' est
• un groupe fonctionnel choisi parmi les groupes hydroxy, carbalcoxy dans lequel le groupe alcoxy est en C₁-C4, carboxy, carbamoyle ; ou
• un groupe O-R, R étant un groupe (C₁-C₄)alkyle ou (C₂-C₄)alcényle substitué par un groupe fonctionnel choisi parmi les groupes carbalcoxy dans lequel le groupe alcoxy est en C₁-C₄ et carboxy ;
. R'' est l'hydrogène ;
. W est une liaison directe ; et
. Z est un (C₁-C₄)alkyle.

7. Utilisation selon la revendication 6, caractérisée en ce que l'analogue de phényléthanolamine a la formule I indiquée dans la revendication 1, dans laquelle
- A est un groupe phényle, 3-chlorophényle ou 3-trifluorométhyphényle ;
- X est l'hydrogène,
- Y est l'hydrogène ou un groupe A'-CH-(OH)-CH₂- où A', identique à A, est phényle ; et
- T représente un groupe de formule II dans laquelle
. n est 1 ou 2 ;
. R' est un groupe hydroxy, carbométhoxy, carbéthoxy, carboxy, carbamoyle, carboxyméthoxy, carbométhoxyméthoxy ou carbéthoxyméthoxy ;
. R'' est l'hydrogène ;
. W est une liaison directe ; et
. Z est méthyle.

8. Utilisation selon la revendication 7, caractérisée en ce que l'analogue de phényléthanolamine est choisi parmi les composés ci- après :
- N-[2-(4-carbométhoxyphényl)-1-méthyléthyl]-2-hydroxy-2-phényléthylamine ;
- N-[2-(4-carboxyphényl)-1-méthyléthyl]-2-hydroxy-2-phényléthylamine ;
- (R,S)-N-(2-phényl-2-hydroxyéthyl)-1-méthyl-3-(4-hydroxyphényl)-propylamine ;
- (R,S)-N-(2-phényl-2-hydroxyéthyl)-1-méthyl-3-(4-aminocarbonylphényl)propylamine ;
- p-[(R)-3-[bis-[(R)-bêta-hydroxyphénéthyl]amino]butyl]benzamide ;
- (RR,SS)-N-[2-(4-carbométhoxyphényl)-1-méthyléthyl]-2-hydroxy-2-(3-trifluorométhylphényl)éthanamine ;
- (RR,SS)-N-[2-(4-carboxyphényl)-1-méthyléthyl]-2-hydroxy-2-(3-trifluorométhylphényl)éthanamine ;
- N-[2-(4-carbométhoxyméthoxyphényl)-1-méthyléthyl]-2-hydroxy-2-(3-chlorophényl)éthanamine ;
- p-[(S)-3-[bis-[(R)-bêta-hydroxyphénéthyl]amino]butyl]-benzamide ;
- N-[2-(4-carboxyméthoxyphényl)-1-méthyléthyl]-2-hydroxy-2-(3-chlorophényl)éthanamine ;
ou un de leurs sels pharmaceutiquement acceptables.

9. Utilisation selon la revendication 8, caractérisée en ce que l'analogue de phényléthanolamine est le fumarate neutre de N-[2-(4-carbométhoxyphényl)-1-méthyléthyl]-2-hydroxy-2-phenyléthylamine sous forme de mélange de diastéréoisomères de plus bas point de fusion.

10. Utilisation selon la revendication 8, caractérisée en ce que l'analogue de phényléthanolamine est la (RR,SS)-N-[2-(4-carbométhoxy-méthoxyphényl)-1-méthyléthyl]-2-hydroxy-2-(3-chlorophényl)éthanamine ou son bromhydrate.

11. Utilisation selon l'une quelconque des revendications 1 à 10 pour la préparation de compositions pharmaceutiques destinées au traitement de l'hypertension oculaire et du glaucome.

12. Composition pharmaceutique destinée au traitement des affections oculaires, caractérisée en ce qu'elle est sous forme d'une solution pour l'application ophtalmique contenant de 0,00001 à 1% d'un analogue de phényléthanolamine ou d'un de ses sels pharmaceutiquement acceptables selon l'une quelconque des revendications 1 à 10, en tant que principe actif en mélange avec un excipient pharmaceutique.

13. Composition pharmaceutique selon la revendication 12, caractérisée en ce qu'elle contient de 0,0001 à 0,2 % de principe actif dans un excipient ophtalmique acceptable pour un usage topique.

14. Composition pharmaceutique selon l'une quelconque des revendications 11 à 13 caractérisée en ce qu'elle est destinée au traitement de l'hypertension intraoculaire et du glaucome.

## Claims

1. Use of a phenylethanolamine analog or one of its pharmaceutically acceptable salts for preparing pharmaceutical compositions intended for the treatment of ocular disorders, said phenylethanolamine analog having the formula 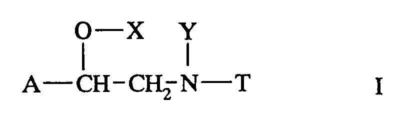 in which
- A represents a benzofuran-2-yl group or a phenyl group, both groups being either unsubstituted or substituted with one or two halogen atoms or with a (C₁-C₄)alkyl or trifluoromethyl group;
- X represents hydrogen, a (C₁-C₄)alkyl or a (C₁-C₄)alkanoyl;
- Y represents hydrogen or a group A'-CH(OH)-CH₂-, A' being identical to A, but different from benzofuran-2-yl; or
- X and Y, teen together, form a methylene group optionally substituted with a carbalkoxy group, in which the alkoxy group is a (C₁-C₄)alkoxy group; an ethylene group optionally substituted with an oxo group; or a 1,3-propylene group;
- T represents a group of formula (II) 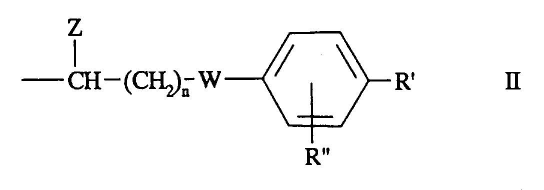 in which
. n is 1, 2, or 3;
. Z is hydrogen or a (C₁-C₄)alkyl group;
. W represents a direct bond or an oxygen atom;
. R' represents
• hydrogen;
• a (C₁-C₄)alkyl group;
• a functional group selected from the following groups: hydroxy; (C₁-C₄)alkoxy; (C₂-C₄)alkenyloxy; (C₂-C₄)alkynyloxy; cycloalkyloxy; cycloalkyl-(C₁-C₄)alkoxy; benzloxy; phenoxy; mercapto; ((C₁-C₄)alkyl)thio; ((C₂-C₄)alkenyl)thio; ((C₂-C₄)alkynyl)thio; cycloalkylthio; (cycloalkyl-(C₁-C₄)alkyl)thio; benzylthio; phenylthio; ((C₁-C₄)alkyl)sulfinyl; ((C₂-C₄)alkenyl)sulfinyl; ((C₂-C₄)alkynyl)sulfinyl; cycloalkylsulfinyl; cycloalkyl-(C₁-C₄)alkyl)sulfinyl; benzylsulfinyl; phenylsulfinyl; ((C₁-C₄)alkyl)sulfonyl; ((C₂-C₄)alkenyl)sulfonyl; ((C₂-C₄)alkynyl)sulfonyl; cycloalkylsulfonyl; (cycloalkyl-(C₁-C₄)alkyl)sulfonyl; benzylsulfonyl; phenylsulfonyl; cyano; nitro; amino either unsubstituted or substituted with one or two radicals which are identical or different and selected from (C₁-C₄)alkyl, (C₂-C₄)alkenyl, (C₂-C₄)alkynyl, cycloalkyl, cycloalkyl-(C₁-C₄)alkyl, benzyl, phenyl; carboxy; carbalkoxy in which the alkoxy group is a C₁-C₄-alkoxy group; ((C₂-C₄)alkenyloxy)carbonyl; ((C₂-C₄)alkynyloxy)carbonyl; cycloalkyloxycarbonyl; (cycloalkyl-C₁-C₄)alkoxy)carbonyl; benzyloxycarbonyl; phenoxycarbonyl; carbamoyl which is unsubstituted or substituted on the amino group with one or two radicals which are identical or different and selected from the (C₁-C₄)alkyl, (C₂-C₄)alkenyl, (C₂-C₄)alkynyl, cycloalkyl, cycloalkyl- (C₁-C₄)alkyl, benzyl and phenyl groups;
• a radical-R selected from the (C₁-C₄)alkyl groups, substituted with a functional group, the (C₂-C₄)alkenyl groups, substituted with a functional group, the (C₂-C₄)alkynyl groups substituted with a functional group, the phenyl((C₁-C₄)alkyl) groups substituted on the phenyl group with a (C₁-C₄)alkyl or a functional group, the phenyl((C₂-C₄)alkenyl) groups, substituted on the phenyl group with a (C₁-C₄)alkyl or a functional group, the phenyl((C₂-C₄)alkynyl) groups substituted on the phenyl group with a (C₁-C₄)alkyl or a functional group, the benzyl groups substituted on the phenyl group with a (C₁-C₄)alkyl or a functional group or the phenyl groups either unsubstituted or substituted with a (C₁-C₄)alkyl or a functional group, the functional group being defined as above;
• a group O-R, S-R, SO-R or SO₂R, in which R is as defined above;
• a group NRR^{·}, where R is as defined above and R^{·} represents hydrogen or is as defined above for R, or R and R^{·} form, with the nitrogen atom to which they are bonded a group selected from the pyrrolidino, piperidino and morpholino groups;
• a COOR, or a CO-SR group, in which R is as defined above;
• a group CONNR^{·}, where R is as defined above and R^{·} represents hydrogen or is as defined above for R, or R and R^{·} form, with the nitrogen atom to which they are bonded, a group selected from the pyrrolidino, piperidino and morpholino groups; and
• a group SO₂NRR^{·}, where R is as defined above and R^{·} represents hydrogen or is as defined above for R, or R and R^{·} form, with the nitrogen atom to which they are bonded, a group selected from the pyrrolidino, piperidino and morpholino groups; and
. R'' represents
• hydrogen;
• a halogen;
• a (C₁-C₄)alkyl;
• a functional group as defined above;
• a group O-R, R being as defined above;
• a group COOR, R being as defined above;
• a group CONRR^{·}, where R is as defined above and R^{·} represents hydrogen or is as defined above for R, or R and R^{·} form, with the nitrogen atom to which they are bonded, a group selected from the pyrrolidino, piperidino and morpholino groups; or
- when A represents a phenyl group, which is unsubstituted or substituted with a halogen atom, a (C₁-C₄)alkyl group or a trifluoromethyl group, and X and Y are hydrogen, T may also represent a group of formula (III) 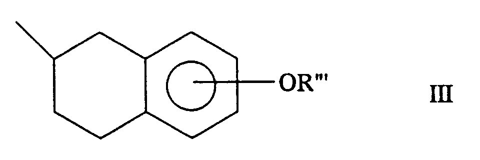 in which -R''' represents hydrogen or a methyl group which is unsubstituted or substituted with a carboxy or carbalkoxy group in which the alkoxy group is a (C₁-C₄)alkoxy group; or an alkyl group having 3 to 5 carbon atoms with a straight or branched chain which is substituted with a carboxy or carbalkoxy group in which the alkoxy group is a (C₁-C₄)alkoxy group.

2. Use according to Claim 1, characterized in that the phenylethanolamine analog has the formula I indicated in Claim 1 in which
- A represents a phenyl group, either unsubstituted or substituted with a halogen atom, a (C₁-C₄)alkyl group or a trifluoromethyl group
- X and Y are hydrogen, and
- T is a group of formula III in which R''' is as defined in Claim 1.

3. Use according to Claim 2, characterized in that the phenylethanolamine analog has formula I indicated in Claim 1, in which
- A is a phenyl group which is unsubstituted or substituted with a halogen atom, a (C₁-C₄)alkyl group or a trifluoromethyl group
- X and Y are hydrogen, and
- T represents a group of formula III in which R''' is hydrogen or a methyl group which is unsubstituted or substituted with a carboxy or carbalkoxy group in which the alkoxy group is a (C₁-C₄)alkoxy group.

4. Use according to Claim 2, characterized in that the phenylethanolamine has the formula I in which A is a 3-chlorophenyl group and R''' is hydrogen, a carbomethoxymethyl, carboethoxymethyl, carbomethoxybutyl, carboethoxybutyl, 2-carbomethoxy-2-propyl or 2-carboethoxy-2-propyl group.

5. Use according to Claim 4, characterized in that the phenylethanolamine is selected from the group consisting of:
- 2-[(7-hydroxy-1,2,3,4-tetrahydronaphth-2-yl)amino]-1-(3-chlorophenyl)ethanol and its pharmaceutically acceptable salts;
- N-[(2S)-7-hydroxy-1,2,3,4-tetrahydronaphth-2-yl]-(2R)-2-(3-chlorophenyl)-2-hydroxyethanamine and its pharmaceutically acceptable salts;
- N-[(2R)-7-hydroxy-1,2,3,4-tetrahydronaphth-2-yl]-(2R)-2-(3-chlorophenyl)-2-hydroxyethanamine and its pharmaceutically acceptable salts;
- N-[(2S)-7-hydroxy-1,2,3,4-tetrahydronaphth-2-yl]-(2S)-2-(3-chlorophenyl)-2-hydroxyethanamine and its pharmaceutically acceptable salts;
- N-[(2R)-7-hydroxy-1,2,3,4-tetrahydronaphth-2-yl]-(2S)-2-(3-chlorophenyl)-2-hydroxyethanamine and its pharmaceutically acceptable salts;
- N-[(2R)-7-ethoxycarbonylmethoxy-1,2,3,4-tetrahydronaphth-2-yl]-(2R)-2-(3-chlorophenyl)-2-hydroxyethanamine and its pharmaceutically acceptable salts;
- N-[(2S)-7-ethoxycarbonylmethoxy-1,2,3,4-tetrahydronaphth-2-yl]-(2R)-2-(3-chlorophenyl)-2-hydroxyethanamine and its pharmaceutically acceptable salts;
- N-[(2S)-7-ethoxycarbonylmethoxy-1,2,3,4-tetrahydronaphth-2-yl]-(2S)-2-(3-chlorophenyl)-2-hydroxyethanamine and its pharmaceutically acceptable salts;
- N-[(2R)-7-methoxycarbonylmethoxy-1,2,3,4-tetrahydronaphth-2-yl]-(2R)-2-(3-chlorophenyl)-2-hydroxyethanamine and its pharmaceutically acceptable salts;
- N-[7-(ethoxycarbonylbutan-4-yloxy)-1,2,3,4-tetrahydronaphth-2-yl]-2-(3-chlorophenyl)-2-hydroryethanamine and its pharmaceutically acceptable salts;
- N-[7-(2-ethoxycarbonylpropan-2-yloxy)-1,2,3,4-tetrahydronaphth-2-yl]-2-(3-chlorophenyl)-2-hydroxyethanamine and its pharmaceutically acceptable salts.

6. Use according to Claim 1, characterized in that the phenylethanolamine analog has the formula I indicated in Claim 1 in which
- A is a phenyl, 2-fluorophenyl, 3-chlorophenyl, or 3-trifluoromethylphenyl group;
- X is hydrogen;
- Y is hydrogen or a group A'-CH(OH)-CH₂- where A' is a phenyl, 2-fluorophenyl, 3-chlorophenyl, or 3-trifluoromethylphenyl group which is identical to A; and
- T is a group of formula II in which
. n is 1 or 2;
. R' is
• a functional group selected from the hydroxy, carbalkoxy in which the alkoxy group is a (C₁-C₄)alkoxy group, carboxy, carbamoyl groups;
or
• a group O-R, R being a (C₁-C₄)alkyl or a (C₂-C₄)alkenyl group which is substituted with a functional group selected from the carbalkoxy groups in which the alkoxy group is a (C₁-C₄)alkoxy group and carboxy;
. R'' is hydrogen;
. W is a direct bond; and
. Z is a (C₁-C₄)alkyl.

7. Use according to Claim 6, characterized in that the phenylethanolamine analog has the formula I indicated in Claim 1 in which
- A is a phenyl, 3-chlorophenyl, or a trifluoromethylphenyl group;
- X is hydrogen,
- Y is hydrogen or a group A'-CH-(OH)-CH₂- where A', identical to A, is phenyl; and
- T is a group of formula III in which
. n is 1 or 2;
. R' is a hydroxy, carbomethoxy, carbethoxy, carboxy, carbamoyl, carboxymethoxy, carbomethoxymethoxy or carbethoxymethoxy group;
. R'' is hydrogen
. W is a direct bond; and
. Z is methyl.

8. Use according to Claim 7, characterized in that the phenylethanolamine analog is selected from the following compounds:
- N-[2-(4-carbomethoxyphenyl)-1-methylethyl]-2-hydroxy-2-phenylethylamine;
- N-[2-4-carboxyphenyl)-1-methylethyl]-2(hydroxy-2-phenylethylamine;
- (R,S)-N-(2-phenyl-2-hydroxyethyl)-1-methyl-3-(4-hydroxyphenyl)propylamine;
- (R,S)-N-(2-phenyl-2-hydroxyethyl)-1-methyl-3-(4-aminocarbonylphenyl)propylamine;
- p-[(R)-3-[bis-[(R)-beta-hydroxyphenethyl]amino]butyl]benzamide;
- (RR,SS)-N-[2-(4-carbomethoxyphenyl)-1-methylethyl]-2-hydroxy-2-(3-trifluoromethylphenyl)ethanamine;
- (RR,SS)-N-[2-(4-carboxyphenyl)-1-methylethyl]-2-hydroxy-2-(3-trifluoromethylphenyl)ethanamine;
- N-[2-(4-carbomethoxymethoxyphenyl)-1-methylethyl]-2-hydroxy-2-(3-chlorophenyl)ethanamine;
- p-[(S)-3-[bis-[(R)-beta-hydroxyphenethyl]amino]butyl]benzamide;
- N-[2-(4-carboxymethoxyphenyl)-1-methylethyl]-2-hydroxy-2-(3-chlorophenyl)ethanamine;
or one of their pharmaceutically acceptable salts.

9. Use according to Claim 8, characterized in that the phenylethanolamine analog is the lower melting diastereoisomer mixture of N-[2-(4-carbomethoxyphenyl)-1-methylethyl]-2-hydroxy-2-phenylethylamine neutral fumarate.

10. Use according to claim 8, characterized in that the phenylethanolamine analog is (RR,SS)-N-[2-(4-carbomethoxymethoxyphenyl)-1-methylethyl]-2-hydroxy-2-(3-chlorophenyl)ethanamine or its hydrobromide.

11. Use according to any one of Claims 1 to 10 for the preparation of pharmaceutical compositions intended for the treatment of occular hypertension and glaucoma.

12. Pharmaceutical composition intended for the treatment of ocular disorders, characterized in that it is in the form of a solution for ophtalmic application containing from 0.0001 to 1 % of a phenylethanolamine analog or of one of its pharmaceutically acceptable salts according to any one of claims 1 to 10, as the active principle in admixture with a pharmaceutical excipient.

13. Pharmaceutical composition according to claim 12, characterized in that it contains from 0.0001 to 0.2 % of active principle in an ophthalmic excipient acceptable for topical use.

14. Pharmaceutical composition according to any one of Claims 11 to 13, characterized in that it is intended for the treatment of intraocular hypertension and glaucoma.

## Patentansprüche

1. Verwendung eines Phenylethanolamin-Analogons oder eines seiner pharmazeutisch akzeptablen Salze zur Herstellung von pharmazeutischen Zusammensetzungen, die zur Behandlung von Augenerkrankungen bestimmt sind, wobei das Phenylethanolamin-Analogon die Formel 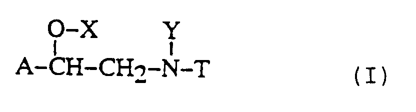 aufweist, worin bedeuten:
- A eine Benzofuran-2-yl-Gruppe oder eine Phenylgruppe, die gegebenenfalls mit einem oder zwei Halogenatomen oder einer C₁₋₄-Alkylgruppe oder einer Trifluormethylgruppe substituiert sind,
- X Wasserstoff, C₁₋₄-Alkyl oder C₁₋₄-Alkanoyl,
- Y Wasserstoff oder eine Gruppe A'-CH(OH)-CH₂-, wobei A' identisch mit A ist, aber nicht Benzofuran-2-yl bedeutet,
oder
X und Y zusammen eine Methylengruppe, die ggfs. mit einer Carbalkoxygruppe substituiert ist, wobei die Alkoxygruppe 1 bis 4 Kohlenstoffatome aufweist, eine Ethylengruppe, die ggfs. mit einer Oxo-Gruppe substituiert ist, oder eine 1,3-Propylengruppe,
- T eine Gruppe der Formel II 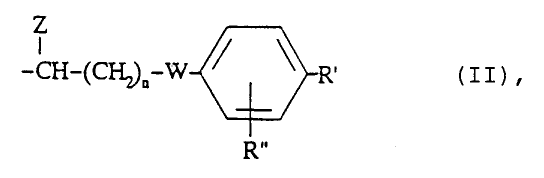 worin bedeuten:
. n 1, 2 oder 3;
. Z Wasserstoff oder C₁₋₄-Alkyl;
. W eine Einfachbindung oder ein Sauerstoffatom;
. R'
• Wasserstoff;
• C₁₋₄-Alkyl
• eine funktionelle Gruppe, die unter folgenden Gruppen ausgewählt ist: Hydroxy; C₁₋₄-Alkoxy; C₂₋₄-Alkenyloxy; C₂₋₄-Alkinyloxy; Cycloalkyloxy; Cycloalkyl-C₁₋₄-alkoxy; Benzyloxy; Phenoxy; Mercapto; C₁₋₄₋Alkylthio; C₂₋₄-Alkenylthio; C₂₋₄-Alkinylthio; Cycloalkylthio; Cycloalkyl-C₁₋₄-alkylthio; Benzylthio; Phenylthio; C₁₋₄-Alkyl-sulfinyl; C₂₋₄-Alkenylsulfinyl; C₂₋₄-Alkinylsulfinyl; Cycloalkylsulfinyl; Cycloalkyl-C₁₋₄₋alkylsulfinyl; Benzylsulfinyl, Phenylsulfinyl; C₁₋₄-Alkylsulfonyl; C₂₋₄-Alkenylsulfonyl; C₂₋₄-Alkinylsulfonyl; Cycloalkylsulfonyl; Cycloalkyl-C₁₋₄-alkylsulfonyl; Benzylsulfonyl; Phenylsulfonyl; Cyano; Nitro; Amino, das unsubstituiert oder mit einer oder zwei Gruppen substituiert ist, die identisch oder verschieden sind und unter C₁₋₄-Alkyl, C₂₋₄-Alkenyl, C₂₋₄-Alkinyl, Cycloalkyl, Cycloalkyl-C₁₋₄-alkyl, Benzyl und Phenyl, ausgewählt sind, Carboxy, Carbalkoxy, wobei die Alkoxygruppe 1 bis 4 Kohlenstoffatome aufweist; C₂₋₄-Alkenyloxycarbonyl; C₂₋₄-Alkinyloxycarbonyl; Cycloalkyloxycarbonyl; Cycloalkyl-C₁₋₄-alkoxycarbonyl; Benzyloxycarbonyl; Phenoxycarbonyl; Carbamoyl, das unsubstituiert oder an der Aminogruppe mit einer oder zwei Gruppen substituiert ist, die unter C₁₋₄-Alkyl, C₂₋₄-Alkenyl, C₂₋₄-Alkinyl, Cycloalkyl, Cycloalkyl-C₁₋₄-alkyl, Benzyl und Phenyl ausgewählt sind;
• eine Gruppe R, die ausgewählt ist unter: C₁₋₄-Alkylgruppen, die mit einer funktionellen Gruppe substituiert sind, C₂₋₄-Alkenylgruppen, die mit einer funktionellen Gruppe substituiert sind, C₂₋₄-Alkinylgruppen, die mit einer funktionellen Gruppe substituiert sind, Phenyl-C₁₋₄-alkyl-Gruppen, die an der Phenylgruppe mit C₁₋₄-Alkyl oder einer funktionellen Gruppe substituiert sind, Phenyl-C₂₋₄-alkenyl-Gruppen, die an der Phenylgruppe mit C₁₋₄-Alkyl oder einer funktionellen Gruppe substituiert sind, Phenyl-C₂₋₄-alkinyl-Gruppen, die an der Phenylgruppe mit C₁₋₄-Alkyl oder einer funktionellen Gruppe substituiert sind, Benzylgruppen, die an der Phenylgruppe mit C₁₋₄-Alkyl oder einer funktionellen Gruppe substituiert sind, oder Phenylgruppen, die unsubstituiert oder mit C₁₋₄-Alkyl oder einer funktionellen Gruppe substituiert sind, wobei die funktionelle Gruppe die oben definierte Bedeutung aufweist;
• eine Gruppe O-R, S-R, SO-R oder SO₂R, wobei R wie oben definiert ist;
• eine Gruppe NRR^{·}, wobei R wie oben definiert ist und R^{·} Wasserstoff bedeutet oder die oben definierte Bedeutung von R aufweist oder R und R^{·} mit dem Stickstoffatom, an das sie gebunden sind, eine Gruppe bilden, die unter Pyrrolidino, Piperidino und Morpholino ausgewählt ist;
• eine Gruppe COOR oder eine Gruppe CO-SR, wobei R wie oben definiert ist;
• eine Gruppe CONRR^{·}, wobei R wie oben definiert ist und R^{·} Wasserstoff bedeutet oder die oben definierte Bedeutung für R aufweist oder R und R^{·} mit dem Stickstoffatom, an das sie gebunden sind, eine Gruppe bilden, die unter Pyrrolidino, Piperidino und Morpholino ausgewählt ist;
oder
• eine Gruppe SO₂NRR^{·}, wobei R wie oben definiert ist und R^{·} Wasserstoff bedeutet oder die oben definierte Bedeutung für R aufweist oder R und R^{·} mit dem Stickstoffatom, an das sie gebunden sind, eine Gruppe bilden, die unter Pyrrolidino, Piperidino und Morpholino ausgewählt ist,
und
. R''
• Wasserstoff;
• Halogen;
• C₁₋₄-Alkyl;
• eine der oben definierten funktionellen Gruppen;
• eine Gruppe O-R, wobei R die oben definierte Bedeutung aufweist;
• eine Gruppe COOR, wobei R die oben definierte Bedeutung aufweist;
• eine Gruppe CONRR^{·}, wobei R wie oben definiert ist und R^{·} Wasserstoff bedeutet oder die oben definierte Bedeutung für R aufweist oder R und R^{·} mit dem Stickstoffatom, an das gebunden sind, eine Gruppe bilden, die unter Pyrrolidino, Piperidino und Morpholino ausgewählt ist,
- oder T kann ferner, wenn A eine Phenylgruppe bedeutet, die gegebenenfalls mit einem Halogenatom einer C₁₋₄-Alkylgruppe oder einer Trifluormethylgruppe substituiert ist, und X und Y Wasserstoff bedeuten, eine Gruppe der Formel III 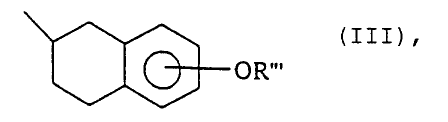
worin
R''' Wasserstoff, eine ggf. mit einer Carboxy- oder Carbalkoxygruppe substituierte Methylgruppe, wobei die Alkoxygruppe 1 bis 4 Kohlenstoffatome aufweist, oder eine geradkettige oder verzweigte Alkylgruppe mit 3 bis 5 Kohlenstoffatomen, die mit einer Carboxy- oder Carbalkoxygruppe substituiert ist, wobei die Alkoxygruppe 1 bis 4 Kohlenstoffatome aufweist, bedeutet.

2. Verwendung nach Anspruch 1, dadurch gekennzeichnet, daß das Phenylethanolamin-Analogon die in Anspruch 1 angebene Formel I aufweist, worin bedeuten:
- A eine Phenylgruppe, die ggf. mit einem Halogenatom, einer C₁₋₄-Alkylgruppe oder einer Trifluormethylgruppe substituiert ist,
- X und Y Wasserstoff und
- T eine Gruppe der Formel III, worin R''' wie in Anspruch 1 definiert ist.

3. Verwendung nach Anspruch 2, dadurch gekennzeichnet, daß das Phenylethanolamin-Analogon die in Anspruch 1 angegebene Formel I aufweist, worin bedeuten:
- A eine Phenylgruppe, die ggf. mit einem Halogenatom, einer C₁₋₄-Alkylgruppe oder einer Trifluormethylgruppe substituiert ist,
- X und Y Wasserstoff
und
- T eine Gruppe der Formel III, in der R''' Wasserstoff oder eine Methylgruppe bedeutet, die ggf. mit einer Carboxy- oder Carbalkoxy-Gruppe substituiert ist, wobei die Alkoxygruppe 1 bis 4 Kohlenstoffatome aufweist.

4. Verwendung nach Anspruch 2, dadurch gekennzeichnet, daß das Phenylethanolamin die Formel I aufweist, worin A 3-Chlorphenyl und R''' Wasserstoff, Carbomethoxymethyl, Carboethoxymethyl, Carbomethoxybutyl, Carboethoxybutyl, 2-Carbomethoxy-2-propyl oder 2-Carboethoxy-2-propyl bedeuten.

5. Verwendung nach Anspruch 4, dadurch gekennzeichnet, daß das Phenylethanolamin ausgewählt ist unter:
- 2-[(7-Hydroxy-1,2,3,4-tetrahydronaphth-2-yl)-amino]-1-(3-chlorphenyl)-ethanol und seinen pharmazeutisch akzeptablen Salzen;
- N-[(2S)-7-Hydroxy-1,2,3,4-tetrahydronaphth-2-yl]-(2R)-2-(3-chlorphenyl)-2-hydroxyethylamin und seinen pharmazeutisch akzeptablen Salzen;
- N-[(2R)-7-Hydroxy-1,2,3,4-tetrahydronaphth-2-yl]-(2R)-2-(3-chlorphenyl)-2-hydroxyethylamin und seinen pharmazeutisch akzeptablen Salzen;
- N-[(2S)-7-Hydroxy-1,2,3,4-tetrahydronaphth-2-yl]-(2S)-2-(3-chlorphenyl)-2-hydroxyethylamin und seinen pharmazeutisch akzeptablen Salzen;
- N-((2R)-7-Hydroxy-1,2,3,4-tetrahydronaphth-2-yl]-(2S)-2-(3-chlorphenyl)-2-hydroxyethylamin und seinen pharmazeutisch akzeptablen Salzen;
- N-[(2R)-7-Ethoxycarbonylmethoxy-1,2,3,4-tetrahydronaphth-2-yl]-(2R)-2-(3-chlorphenyl)-2-hydroxyethylamin und seinen pharmazeutisch akzeptablen Salzen;
- N-[(2S)-7-Ethoxycarbonylmethoxy-1,2,3,4-tetrahydronaphth-2-yl)-(2R)-2-(3-chlorphenyl)-2-hydroxyethylamin und seinen pharmazeutisch akzeptablen Salzen;
- N-[(2S)-7-Ethoxycarbonylmethoxy-1,2,3,4-tetrahydronaphth-2-yl]-(2S)-2-(3-chlorphenyl)-2-hydroxyethylamin und seinen pharmazeutisch akzeptablen Salzen;
- N-[(2R)-7-Methoxycarbonylmethoxy-1,2,3,4-tetrahydronaphth-2-yl]-(2R)-2-(3-chlorphenyl)-2-hydroxyethylamin und seinen pharmazeutisch akzeptablen Salzen;
- N-[7-(Ethoxycarbonylbutan-4-yloxy)-1,2,3,4-tetrahydronaphth-2-yl]-2-(3-chlorphenyl)-2-hydroxyethyl-amin und seinen pharmazeutisch akzeptablen Salzen;
- N-[7-(2-Ethoxycarbonylpropan-2-yloxy)-1,2,3,4-tetrahydronaphth-2-yl]-2-(3-chlorphenyl)-2-hydroxyethylamin und seinen pharmazeutisch akzeptablen Salzen.

6. Verwendung nach Anspruch 1, dadurch gekennzeichnet, daß das Phenylethanolamin-Analogon die in Anspruch 1 angegebene Formel I aufweist, worin bedeuten:
- A Phenyl, 2-Fluorphenyl, 3-Chlorphenyl oder 3-Trifluormethylphenyl;
- X Wasserstoff;
- Y Wasserstoff oder eine Gruppe A'-CH(OH)-CH₂-, wobei A' identisch mit A ist und Phenyl, 2-Fluorphenyl, 3-Chlorphenyl oder 3-Trifluormethylphenyl bedeutet;
und
- T eine Gruppe der Formel II, worin bedeuten:
. n 1 oder 2;
. R'
• eine funktionelle Gruppe, die unter Hydroxy, Carbalkoxy, wobei die Alkoxygruppe 1 bis 4 Kohlenstoffatome aufweist, Carboxy und Carbamoyl ausgewählt ist,
oder
• eine Gruppe O-R, wobei R eine C₁₋₄-Alkylgruppe oder eine C₂₋₄-Alkenylgruppe, die mit einer funktionellen Gruppe substituiert ist, die unter Carbalkoxy, wobei die Alkoxygruppe 1 bis 4 Kohlenstoffatome aufweist, und Carboxy ausgewählt ist;
. R'' Wasserstoff;
. W eine Einfachbindung
und
. Z C₁₋₄-Alkyl.

7. Verwendung nach Anspruch 6, dadurch gekennzeichnet, daß das Phenylethanolamin-Analogon die in Anspruch 1 angegebene Formel I aufweist, worin bedeuten:
- A Phenyl, 3-Chlorphenyl oder 3-Trifluormethylphenyl;
- X Wasserstoff;
- Y Wasserstoff oder eine Gruppe A'-CH-(OH)-CH₂-, wobei A' mit A identisch ist und Phenyl bedeutet,
und
- T eine Gruppe der Formel II, worin bedeuten:
. n 1 oder 2;
. R' Hydroxy, Carbomethoxy, Carbethoxy, Carboxy, Carbamoyl, Carboxymethoxy, Carbomethoxmethoxy oder Carboethoxymethoxy;
. R'' Wasserstoff;
. W eine Einfachbindung und
. Z Methyl.

8. Verwendung nach Anspruch 7, dadurch gekennzeichnet, daß das Phenylethanolamin-Analogon unter folgenden Verbindungen ausgewählt ist:
- N-[2-(4-Carbomethoxyphenyl)-1-methylethyl]-2-hydroxy-2-phenylethylamin;
- N-[2-(4-Carboxyphenyl)-1-methylethyl]-2-hydroxy-2-phenylethylamin;
- (R,S)-N-(2-Phenyl-2-hydroxyethyl)-1-methyl-3-(4-hydroxyphenyl)-propylamin;
- (R,S)-N-(2-Phenyl-2-hydroxyethyl)-1-methyl-3-(4-aminocarbonylphenyl)-propylamin;
- p-[(R)-3-[Bis[(R)-β-hydroxyphenethyl]amino]-butyl]-benzamid;
- (RR,SS)-N-[2-(4-Carbomethoxvphenyl)-1-methylethyl]-2-hydroxy-2-(3-trifluormethylphenyl)-ethylamin;
- (RR,SS)-N-[2-(4-Carboxvphenyl)-1-methylethyl]-2-hydroxy-2-(3-trifluormethylphenyl)-ethylamin;
- N-(2-(4-Carbomethoxymethoxyphenyl)-1-methylethyl]-2-hydroxy-2-(3-chlorphenyl)-ethylamin;
- p-[(S)-3-[Bis[(R)-β-hydroxyphenethyl]amino]-butyl]-benzamid;
- N-[2-(4-Carboxvmethoxyphenyl)-1-methylethyl]-2-hydroxy-2-(3-chlorphenyl)-ethylamin;
oder
einem ihrer pharmazeutisch akzeptablen Salze.

9. Verwendung nach Anspruch 8, dadurch gekennzeichnet, daß das Phenylethanolamin-Analogon das neutrale Fumarat von N-[2-(4-Carbomethoxyphenyl)-1-methylethyl]-2-hydroxy-2-phenylethylamin in Form des Gemisches der Diastereomeren mit dem niedrigsten Schmelzpunkt ist.

10. Verwendung nach Anspruch 8, dadurch gekennzeichnet, daß das Phenylethanolamin-Analogon das (RR,SS)-N-[2-(4-CarbomethoxXethoxyphenyl)-1-methylethyl]-2-hydroxy-2-(3-chlorphenyl)-ethylamin oder sein Hydrobromid ist.

11. Verwendung nach einem der Ansprüche 1 bis 10 zur Herstellung von pharmazeutischen Zusammensetzungen, die zur Behandlung von erhöhtem Augeninnendruck oder des Glaukoms bestimmt sind.

12. Pharmazeutische Zusammensetzung, die zur Behandlung von Augenerkrankungen bestimmt ist, dadurch gekennzeichnet, daß sie in Form einer Lösung zur ophthalmischen Anwendung vorliegt und 0,00001 bis 1 % eines Phenylethanolamin-Analogons oder eines seiner pharmazeutisch akzeptablen Salze nach einem der Ansprüche 1 bis 10 als Wirkstoff im Gemisch mit einem pharmazeutischen Excipiens enthält.

13. Pharmazeutische Zusammensetzung nach Anspruch 12, dadurch gekennzeichnet, daß sie 0,0001 bis 0,2 % Wirkstoff in einem ophthalmischen, zur topischen Anwendung geeigneten Excipiens enthält.

14. Pharmazeutische Zusammensetzung nach einem der Ansprüche 11 bis 13, dadurch gekennzeichnet, daß sie zur Behandlung von erhöhtem Augeninnendruck oder des Glaukoms bestimmt ist.
